(19) Europäisches Patentamt

European Patent Office

Office européen des brevets

(11) **EP 1 722 345 A1**

(12) **EUROPEAN PATENT APPLICATION**

(43) Date of publication:
**15.11.2006 Bulletin 2006/46**

(51) Int Cl.:
*G09B 23/28* (2006.01)     *G06F 17/50* (2006.01)
*G06F 19/00* (2006.01)

(21) Application number: **06009780.5**

(22) Date of filing: **11.05.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **11.05.2005 JP 2005138563**

(71) Applicant: **SYSMEX CORPORATION**
**Kobe-shi,**
**Hyogo 651-0073 (JP)**

(72) Inventors:
• **Kouchi, Yasuhiro**
**Sysmex Corporation**
**Kobe-shi**
**Hyogo 651-0073 (JP)**

• **Saitou, Takeo**
**Sysmex Corporation**
**Kobe-shi**
**Hyogo 651-0073 (JP)**
• **Seike, Masayoshi**
**Sysmex Corporation**
**Kobe-shi**
**Hyogo 651-0073 (JP)**

(74) Representative: **HOFFMANN EITLE**
**Patent- und Rechtsanwälte**
**Arabellastrasse 4**
**81925 München (DE)**

(54) **Biological simulation system and computer program product**

(57) With an object to seek parameters of a biological model corresponding to individual patents, the present invention provides a biological simulation system that comprises an internal parameter set generating section which generates internal parameter sets constituting a biological model, a biological model computing section computing output of a biological model which emulates a biological response of a biological organ based on the generated internal parameter set, and a template database having a plurality of combinations of a reference output value of the biological model and an internal parameter set corresponding to the reference output value, wherein the internal parameter set generating section comprises a database referring means which selects a reference output approximating an actual biological response from said template database and which selects an internal parameter set corresponding to the selected reference output value.

Fig. 1

**Description**

**TECHNICAL FIELD**

The present invention relates to a biological simulation system, particularly a system for simulating pathological condition of diabetes.

**BACKGROUND**

Biological bodies have been conventionally tried to describe by mathematical models. The minimal model by Bergman can be referred to for this model. Bergman's minimal model was disclosed in "American Journal of Physiology, 1979, Vol. 236-6, p.E-667-77, Bergman et al." and "Journal of Clinical Investigation, 1981, Vol. 68-6, p.1456-67".
In this minimal model, variables are blood glucose level, plasma insulin concentration, and insulin action level i.e. remote insulin of insulin action point of a peripheral tissue. The equations of the minimal model are as follows:

$$dG(t) / dt = -p_1(G(t) - G_b) - X(t)G(t)$$

$$dX(t) / dt = -p_2 X(t) + p_3(I(t) - I_b)$$

$$dI(t) / dt = -n(I(t) - I_b) + \gamma(G(t) - h)$$
$$(G(t) > h)$$
$$= -n(I(t) - I_b) + \gamma(G(t) - h)$$
$$(G(t) <= h)$$

where blood glucose level for time "t" is represented by "G(t)", plasma insulin concentration is "I(t)", and remote insulin is "X(t)", and time difference is on the left sides. Parameters in the equation are:

$P_1$ : insulin-independent glucose metabolism rate
$G_b$ : basal blood-glucose level
$p_2$ : insulin incorporation capacity
$p_3$ : insulin consumption rate against insulin-dependent glucose metabolism
$I_b$ : basal insulin concentration
n : insulin consumption per unit time
$\gamma$ : insulin secretion sensitivity against glucose stimulation,
h : threshold level of blood glucose starting insulin secretion.

These values depend on individuals.
If we try to simulate a biological body by applying such model to an individual patient and use the model for diagnosis and the like, we need to appropriately set the above-mentioned parameters constituting the biological model depending on the individual patients.
That means, when we try to reproduce an actual patient body by the biological model, we need accuracy of the above-mentioned parameters and obtain accurate parameters different among individual patients as much as possible.

**SUMMARY**

Therefore, an object of the present invention is to provide a technical means for obtaining parameters of biological models corresponding to individual patients.
A first invention is a biological simulation system using a biological model comprising an internal parameter set generating section generating internal parameter sets constituting a biological model, a biological model computing section computing output of a biological model which emulates a biological response of a biological organ based on the internal parameter set, and a template database having a plurality of combinations of a reference output value of the biological model and an internal parameter set corresponding to the reference output value, wherein said internal parameter set generating section comprises a database reference means which selects a reference output value approximate to an actual biological response from said template database and which selects an internal parameter set corresponding to the selected reference output value.

A second invention is a biological simulation system using a biological model comprising an internal parameter set generating section generating internal parameter sets constituting a biological model, a biological model computing section emulating a biological response of the biological organ based on the internal parameter set, and a template database having a plurality of combinations of a reference output value of the biological model and a search range of an internal parameter set corresponding to the reference output value,

wherein said internal parameter set generating section comprises: a database reference means which selects a reference output value approximate to an actual biological response from said template database and which selects the search range of the internal parameter set corresponding to the selected reference output value; a means for automatically generating a plurality of different internal parameter sets within said search range; and a selecting means which determines an approximation between a biological model output calculated applying the automatically generated internal parameter set and an actual biological response and which selects an appropriate internal parameter set from a plurality of the generated internal parameter sets.

A third invention is a biological simulation system using a biological model comprising an internal parameter set generating section generating internal parameter sets constituting a biological model, a biological model computing section emulating a biological response of the biological organ based on the internal parameter set, and a template database having a plurality of combinations of a reference output value of the biological model and a selection range of an internal parameter set corresponding to the reference output value,

wherein said internal parameter set generating section comprises: a database reference means which selects a reference output approximate to an actual biological response from said template database and which selects the selection range of the internal parameter set corresponding to the selected reference output value; a means for automatically generating a plurality of different internal parameter sets within said selection range; a first selecting means which determines an approximation between a biological model output calculated applying the automatically generated internal parameter set and an actual biological response and which selects an appropriate internal parameter set from a plurality of the generated internal parameter sets, and a second selecting means for the selecting parameter within said selection range from the internal parameter sets selected by said first selecting means.

Further, with regard to an invention related to a computer program product, a computer is executed to perform the biological simulation as the biological simulation system.

## DESCRIPTION OF DRAWINGS

FIG. 1 is a block diagram showing a hardware construction of a system of the present invention.
FIG. 2 is a block diagram showing overall construction of a biological model.
FIG. 3 is a block diagram showing a construction of pancreas model of the biological model.
FIG. 4 is a block diagram showing a construction of a hepatic metabolism model of the biological model.
FIG. 5 is a block diagram showing a construction of insulin kinetics model.
FIG. 6 is a block diagram showing a construction of a peripheral tissue model.
FIG. 7 is a flowchart showing procedure of an internal parameter generating section related to a first embodiment.
FIG. 8 is a OGTT time-series datum, (a) is a blood-glucose level, and (b) is a blood-insulin concentration.
FIG. 9 is a construction diagram of a template database DB1.
FIG. 10 is a template database (a) is a blood glucose level and (b) is insulin concentration.
FIG. 11 is diagrams showing an error sum of OGTT time-series data against template T1. (a) is a blood glucose level (b) is an insulin concentration.
FIG. 12 shows biological function profiles.
FIG. 13 is a flowchart of genetic algorithm.
FIG. 14 is a flowchart showing procedures of an internal parameter generating section related to a second embodiment.
FIG. 15 is a flowchart showing procedures of an internal parameter generating section related to a third embodiment.
FIG. 16 is a flowchart showing procedures of an internal parameter generating section related to a fourth embodiment.
FIG. 17 is a construction diagram showing template database DB2.
FIG. 18 is a flowchart showing procedures of an internal parameter generating section related to a fifth embodiment.
FIG. 19 is a construction diagram showing template database DB3.

FIG. 20 is a flowchart showing procedures of internal parameter generating section related to a sixth embodiment.

**DETAILED DESCRIPTION OF THE PREFERRED EMBODIMENT**

Embodiments of the present invention is described hereinafter with reference to drawings.

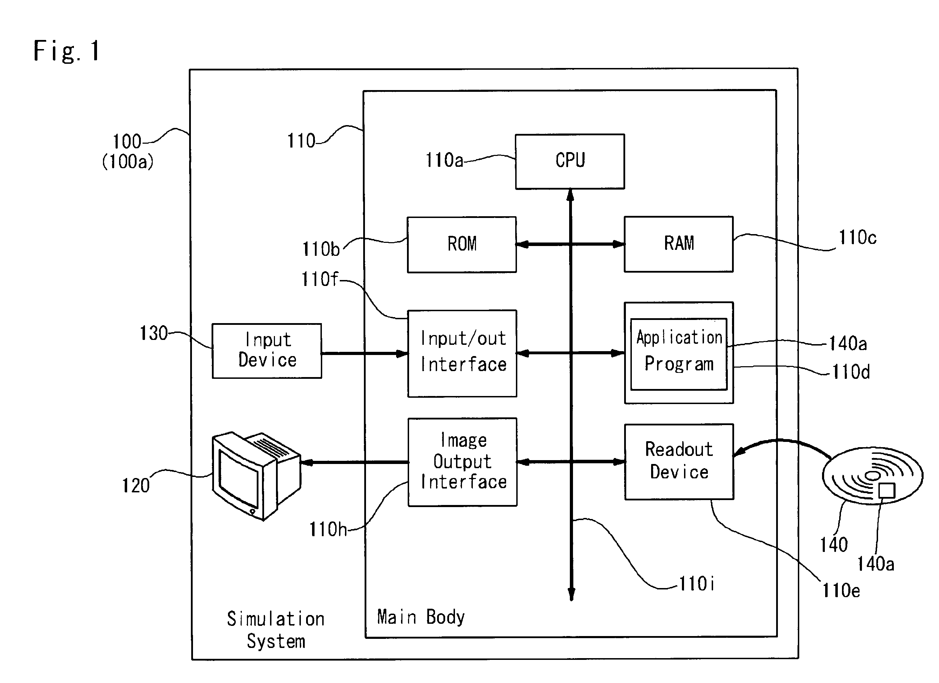

FIG. 1 is a block diagram showing a hardware construction of a biological simulation system (also referred to as "system" hereinafter) related to a first embodiment of the present invention. A system 100 related to the present embodiment is composed of a computer 100a primarily comprising a main body 110, a display 120, and an input device 130. The main body 110 comprises a CPU 110a, a ROM 110b, a RAM 110c, a hard disk 110d, a readout device 110e, an input/output interface 110f, and an image output interface 110h. The CPU 110a, the ROM 110b, the RAM 110c, the hard disk 110d, the readout device 110e, the input/output interface 110f, and the image output interface 110h are data-communicably connected by a bus 110i.

The CPU 110a is capable of executing a computer program recorded in the ROM 110b and a computer program loaded in the RAM 110c. And the CPU 110a executes an application program 140a as described later to realize each function block as described later, thereby the computer 100a functions as the system 100.

The ROM 110b comprises mask ROM, PROM, EPROM, EEPROM, etc. and is recoded with computer programs executed by the CPU 110a and data used for the programs.

The RAM 110c comprises SRAM, DRAM, etc. The RAM 110c is used to read out computer programs recorded in the ROM 110b and the hard disk 110d. And the RAM 110c is used as a work area of the CPU 110a when these computer programs are executed.

The hard disk 110d is installed with an operating system, an application program, etc., various computer programs to be executed by the CPU 110a, and data used for executing the computer programs. An application program 140a described later is also installed in this hard disk 110d.

The readout device 110e which comprises a flexible disk drive, a CD-ROM drive or DVD-ROM drive is capable of reading out a computer program or data recorded in a portable recording media 140. And the portable recording media 140 stores the application program 140a to function as a system of the present invention. The computer 100a reads out the application program 140a related to the present invention from the portable recording media 140 and is capable of installing the application program 140a in the hard disk 110d.

In addition to that said application program 140a is provided by the portable recording media 140, said application program 140a may be provided through an electric communication line (wired or wireless) from outside devices which are communicably connected to the computer 100a via said electric communication line. For example, said application program 140a is stored in a hard disk in an internet server computer to which the computer 100a accesses and said application program 140a may be downloaded and installed in the hard disk 110d.

The hard disk 110d is installed with an operating system which provides a graphical user interface environment, e.g. Windows (Registered trademark) manufactured by US Microsoft corp. In the explanation hereinafter, the application program 140a related to this embodiment shall operate on said operating system.

The input/output interface 110f comprises a serial interface, e.g. USB, IEEE1394, RS-232C, etc.; a parallel interface, e.g. SCSI, IDE, IEEE1284, etc.; and an analog interface e.g. D/A converter, A/D converter, etc. The input/output interface 110f is connected to the input device 130 comprising a keyboard and a mouse and users can input data into the computer 100a using the input data device 130.

The image output interface 110h is connected to the display 120 comprising LCD, CRT or the like so that picture signals corresponding to image data provided from the CPU 110a are output to the display 120. The display 120 displays a picture (screen) based on input picture signals.

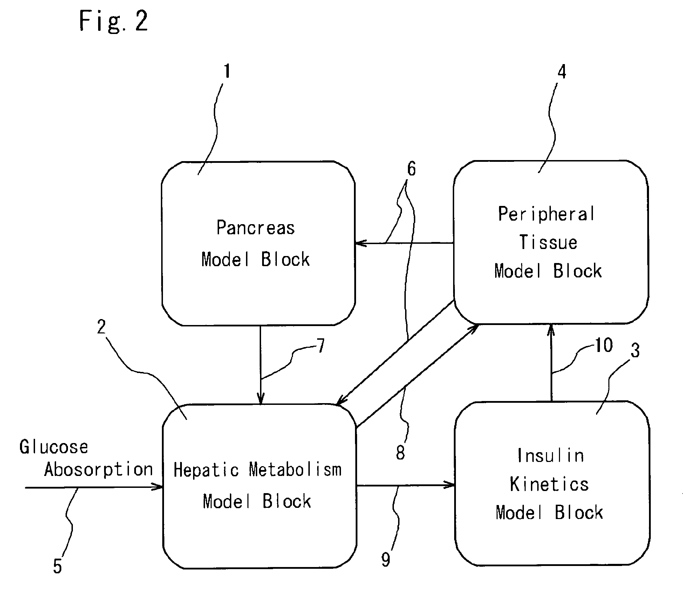

FIG. 2 is a block diagram showing a biological mathematical model (also simply referred to as "biological model" hereinafter) used in the biological simulation system also simply referred to as "system" hereinafter.

As in FIG. 2, the biological model (biological model computing section) comprises a pancreas model block (pancreas model block computing section) 1, a hepatic metabolism model block (hepatic metabolism model block computing section) 2, an insulin kinetics model block (insulin kinetics model block computing section) 3, and a peripheral tissue block (peripheral tissue block computing section) 4, each of which simulates biological organs and has input provided outside the biological model or from other blocks and output to other blocks.

That means, the pancreas model block 1 computes in emulation of a pancreas function. A blood glucose level 6 is set as input and an insulin secretion rate 7 is set as output to other blocks.

The hepatic metabolism model block 2 computes in emulation of a hepatic function. A glucose absorption 5 from digestive tract , a blood glucose level 6 and an insulin secretion rate 7 are set as input and net glucose release 8 and post liver insulin 9 are set as output to other blocks.

The insulin kinetics model block 3 computes in emulation of insulin kinetics. Post liver insulin 9 is set as input and peripheral tissue insulin concentration 10 is set as output to other blocks.

The peripheral tissue block 4 computes in emulation of peripheral tissue function. A net glucose release 8, and insulin concentration 10 in the peripheral tissue are set as input and a blood glucose level 6 is set as output to other blocks.

Said glucose absorption 5 is a data provided from outside and is performed by user inputting inspection data and the like using, for example, the input device 130. Further, the function blocks 1 to 4 are each realized by the CPU 110a executing the computer program 140a.

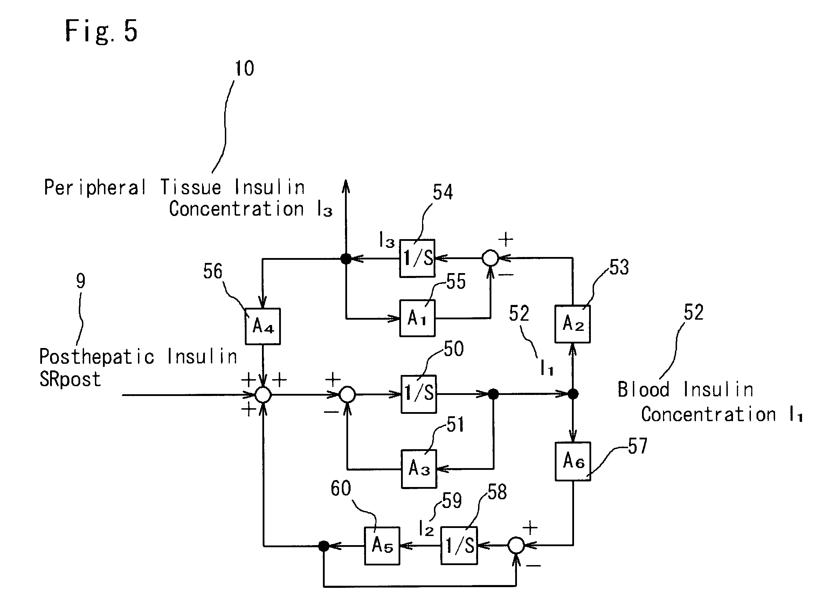

Except for the output to be given to other blocks, there exist values which are calculated in the block 3 but not given to other blocks as they are like the blood-insulin concentration $I_1$ (FIG. 5) in the insulin kinetics model block 3. Such values are also regarded as values obtained from the biological model in terms of the biological model as a whole and the values therefore may be included in the output of the biological model.

Next, the above-mentioned blocks each are described in detail. FGB expresses a fasting blood glucose level (FGB = BG (0)), and Ws expresses an assumed weight. DVg and DVi respectively express a distribution capacity volume against glucose and a distribution capacity volume against insulin.

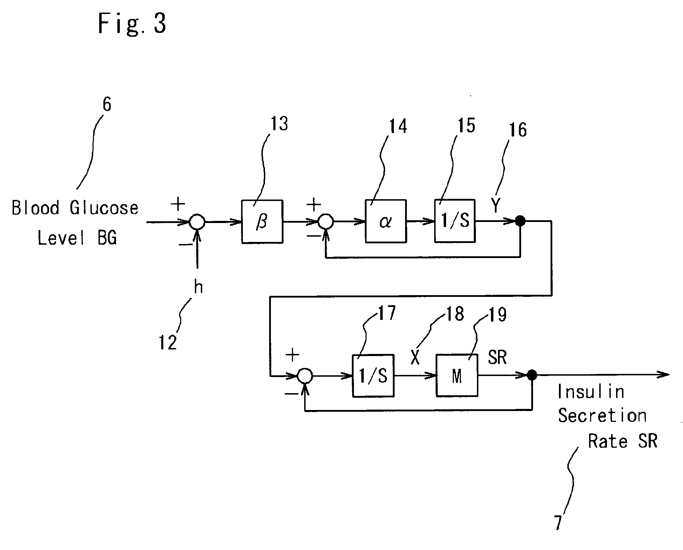

Relationship between input and output of the pancreas model block 1 may be expressed using the following differential equation (1). A block diagram as in FIG. 3 equivalent to the differential equation (1) may be also used.

Differential equation (1):

$$dY/dt = - \alpha \{Y(t) - \beta(BG(t) - h)\} \quad (BG(t) > h)$$
$$= - \alpha Y(t) \qquad (BG(t) <= h)$$
$$dX/dt = - M \cdot X(t) + Y(t)$$
$$SR(t) = M \cdot X(t)$$

Variables:

BG(t): blood glucose level
X(t): total amount of insulin capable of secretion from pancreas
Y(t): supply rate of insulin newly supplied for glucose stimulation
SR(t): pancreas insulin secretion rate Parameters:
h: threshold of glucose concentration capable of stimulating insulin supply
$\alpha$: following performance to glucose stimulation
$\beta$: sensitivity to glucose stimulation
M: secretion rate per unit concentration

where a blood glucose level 6 which is input to the pancreas model block in FIG. 2 corresponds to BG(t). The insulin secretion rate 7 which is output of the pancreas model block in FIG. 2 corresponds to SR(t).

In FIG. 3, numeral 6 indicates a blood glucose level BG : 7, pancreas insulin secretion rate from pancreas SR (t); 12, glucose concentration threshold stimulating insulin supply h; 13, glucose stimulation sensitivity $\beta$; 14, glucose stimulation following capability $\alpha$; 15, integral element; 16, supply rate of newly supplied insulin to glucose stimulation Y(t); 17, integral element; 18, total amount of insulin capable of secretion from pancreas X(t); 19, secretion rate per unit concentration M.

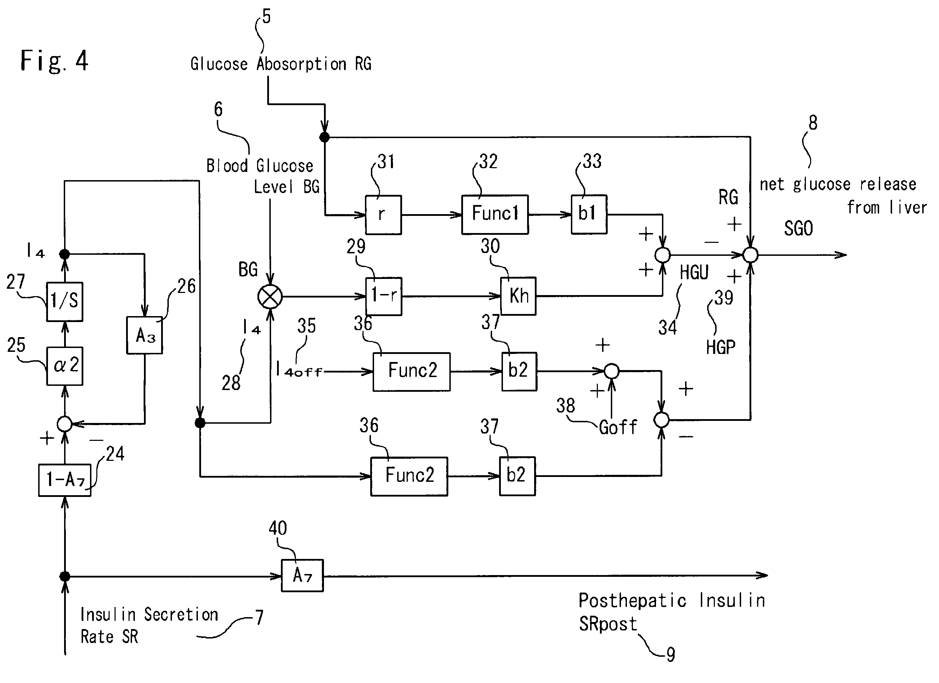

Relationship between input and output of the hepatic metabolism model block 2 may be described using the following differential equation (2). A block diagram as in FIG. 4 equivalent to the differential equation (2) may be also used.

```
Differential equation (2):
    dI₄(t) / dt  = α2 {-A₃I₄(t) + (1-A₇)·SR(t)}
    Goff (FBG)  = f1          (FBG < f3)
                = f1 + f2·(FBG – f3)    (FBG >=f3)
    Func1 (FGB) = f4 – f5·(FBG – f6)
    Func2 (FGB) = f7 / FBG
    b1(I₄(t))   = f8{1 + f9·I₄(t)}
    HGU(t)      = r·Func1(FBG)· b1(I₄(T))·RG(t) +
                  (1-r)·Kh·BG(t)·I₄(t)     (HGU(t)>= 0)
    HGP(t)      = I₄off·Func2(FBG)·b2+G off(FBG)
                  -I4(t)·Func2(FBG)·b2     (HGP(t)>= 0)
    SGO(t)      = RG(t) + HGP(t) – HGU (t)
    SRpost(t)   = A₇SR(t)
```

Variables:

BG(t): blood glucose level
SR(t): pancreas insulin secretion rate
SRpost(t): posthepatic insulin
RG($_t$): glucose absorption from digestive tract
HGP(t): hepatic glucose release
HGU (t): hepatic glucose uptake
SGO (t): net glucose from liver
$I_4$(t): hepatic insulin concentration

Parameter:

Kh: hepatic glucose uptake rate per unit insulin and unit glucose
$A_7$: insulin uptake rate in liver
Goff: glucose release rate to basal metabolism
b2: adjustment term for hepatic glucose release surpression rate
r: insulin-dependent hepatic glucose uptake distribution rate
α2: transmission efficiency to hepatic insulin
$I_{4off}$: insulin concentration threshold of hepatic glucose release surpression

Function:

Goff (FBG): glucose release rate to basal metabolism
Func1(FBG): hepatic glucose uptake rate to stimulation of glucose from digestive tract
Func2 (FBG): hepatic glucose release surpression rate to insulin stimulation
f1 to f9: constants used to express the above-mentioned three elements
b1($I_4$(t)): adjustment item for hepatic glucose incorporation rate

where the glucose absorption from digestive tract 5 which is input to the hepatic metabolism model block in FIG. 2 corresponds to RG(t), the blood glucose level 6 to BG(t) and the insulin secretion rate 7 to SR(t). The net glucose release 8 which is output corresponds to SGO(t) and the posthepatic insulin 9 to SRpost(t).

In FIG. 4, numeral 5 expresses glucose absorption from digestive tract RG(t); 6, blood glucose level BG(t); 7, pancreas insulin secretion rate SR(t); 8, net glucose from liver SGO(t);9, posthepatic insulin SRpost(t); 24, liver insulin passage rate (1 - A7) ; 25, transmission efficiency to hepatic insulin α2; 26, post liver insulin distribution rate A3; 27, integral element; 28, hepatic insulin concentration 14(t); 9, insulin-dependant hepatic glucose incorporation distribution rate (1-r); 30, liver glucose incorporation rate per unit insulin and unit glucose Kh; 32, insulin -independent hepatic glucose incorporation rate r; 32, hepatic glucose incorporation rate to glucose stimulation from digestive tract Funcl (FBG) ; 33, adjustment item for hepatic incorporation rate b1 ($I_4(t)$); 34, hepatic glucose incorporation HGU(t); 35, insulin concentration threshold of hepatic glucose release inhibition $I_{4off}$; 36, hepatic release-inhibition rate to insulin stimulation Func2 (FBG); 37, adjustment items hepatic glucose release -inhibition rate b2; 38, glucose release rate to basal metabolism; 39, hepatic glucose release HGP(t); 40, insulin incorporation rate in liver $A_7$.

Relationship between input and output of the insulin kinetics secretion may be described using the following differential equation (3). A block diagram as in FIG. 5 equivalent to the differential equation (3) may be also used.

Differential equation (3):

$$dI_1(t) / dt = - A_3 I_1(t) + A_5 I_2(t) + A_4 I_3(t) + SRpost(t)$$
$$dI_2(t) / dt = A_6 I_1(t) - A_5 I_2(t)$$
$$dI_3(t) / dt = A_2 I_1(t) - A_1 I_3(t)$$

Variables:

SRpost(t): posthepatic insulin
$I_1(t)$: blood insulin concentration
$I_2(t)$: insulin concentration in insulin-independent tissues
$I_3(t)$: insulin concentration in peripheral tissues Parameters:
$A_1$: disappearance rate in peripheral tissues
$A_2$: insulin distribution rate to peripheral tissues
$A_3$: posthepatic insulin distribution rate
$A_4$: post peripheral tissue insulin flow out rate
$A_5$: insulin disappearance rate in insulin-independent tissues
$A_6$: insulin distribution rate to insulin-independent tissues

where the post liver insulin 9 which is input to the insulin kinetics model block in FIG.b 2 corresponds to SRpost(t). The peripheral tissue insulin concentration 10 which is output corresponds to $I_3(t)$.

In FIG. 5, numeral 9 expresses post liver insulin SRpost (t) ; 10, insulin concentration in peripheral tissue $I_3$ (t) ; 50, integral element; 51, post liver insulin distribution rate $A_3$; 52, blood insulin concentration $I_1(t)$; 53, insulin distribution rate to insulin- independent tissue $A_2$; 54, integral element; 55, insulin disappearance rate in peripheral tissue $A_1$; 56, post peripheral tissue insulin discharge rate $A_4$; 57, insulin distribution rate to insulin-independent tissue $A_6$; 58, integral element; 59, insulin concentration in insulin-independent tissue $I_2(t)$ ; 60, insulin disappearance rate in insulin-independent tissue $A_5$.

Relationship between input and output of the peripheral metabolism model block 4 may be described using the following differential equation (4). A block diagram as in FIG. 4 equivalent to the differential equation (4) may be also used.

Differential equation (4):

$$dBG' / dt = SGO(t) - u*Goff (FBG) - Kb \cdot BG'(t) - Kp \cdot I_3(t) \cdot BG'(t)$$

Variables:

BG' (t): blood glucose level (BG[mg/d1], BG'[mg/kg])
SGO(t): net glucose from liver
$I_3(t)$: insulin concentration in peripheral tissues Parameters:
Kb: insulin-independent glucose consumption rate in peripheral tissues
Kp: insulin-dependent glucose consumption rate in peripheral tissues per unit insulin and per unit glucose
u: ratio of insulin-independent glucose consumption to basal metabolism in glucose release rate to basal metabolism Function
Goff(FBG): glucose release rate to basal metabolism

f1 to f3: constant used to express Goff

where the peripheral tissue insulin concentration 10 which is input to the peripheral metabolism model block in FIG. 2 corresponds to $I_3(t)$, the net glucose 8 from liver corresponds to SGO(t). The blood glucose level 6 which is output corresponds to BG(t).

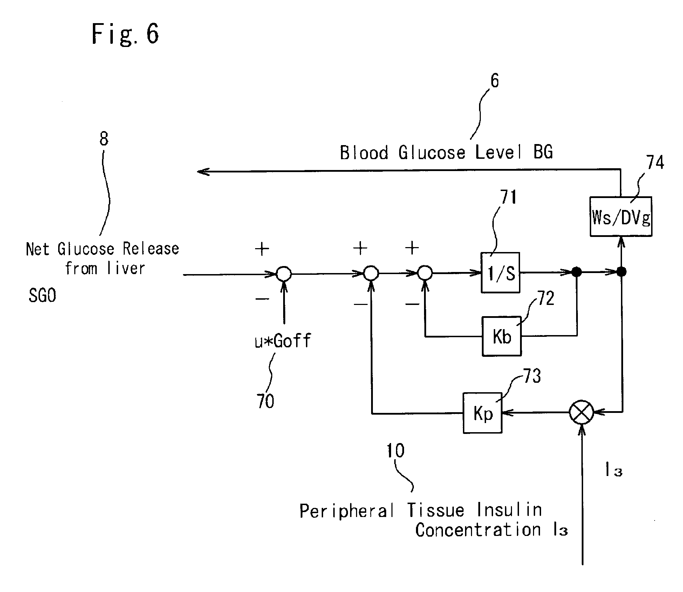

In FIG. 6, numeral 6 expresses blood glucose level BG(t); 8, net glucose from liver SGO(t); 10, insulin concentration in peripheral tissue $I_3(t)$; 70, insulin-independent glucose consumption rate to basal metabolism u*Goff (FBG) ; 71, integral element; 72, insulin-independent glucose consumption rate in peripheral tissue Kb; 73, insulin-dependent glucose consumption rate in peripheral tissue per unit insulin and per unit glucose Kp; 74, unit conversion constant Ws/DVg.

Each block outputs time-series change of each output item based on the above-mentioned differential equation. Further, as in FIG. 2, input/output between blocks constituting the present system is connected to each other and output of a certain block gives input of the other block, so that output of each block changes according to the time-series change of the block output. Therefore, for example, when glucose absorption RG from digestive tract is input in the biological model, time-series change of values of blood glucose level: BG(t) and blood insulin concentration: $I_1(t)$ are calculated and simulated based on the mathematical formulas.

Thus, the blood glucose level and the insulin concentration which have been sequentially calculated in such way can be displayed in the display 120. Thereby users can easily confirm results of the biological organ simulation as mentioned above. Further, it is possible to employ the present system as a subsystem for simulating biological functions in a medical system such as a diabetes diagnosis supporting system. In this case, the time-series change of calculated blood glucose level and insulin concentration is passed to other components of medical systems, by which, for example, diabetes diagnosis supporting information is provided. It is possible to obtain reliable medical information based on the blood glucose level and insulin concentration calculated by the present system.

With regard to calculation of the differential equations of the present system, e. g. E-cell (software disclosed by Keiou University) and MatLab (manufactured by the MathWorks Inc.) may be employed. Or other calculation system may be employed.

To simulate individual patient biological organs using the above-mentioned biological models as shown in FIGs. 2 to 6, it is required to determine an initial value of the above mentioned variables and parameters according to individual patient. (Unless otherwise specified, a variable initial value shall be included in parameters of subjects to be generated hereinafter).

For this purpose, the present system has a parameter set generation function (parameter set generating section) which obtains an internal parameter set of internal parameter group of the biological model (also simply referred to as "parameter set" hereinafter). The parameter set generated by said function is provided to said biological model so that a biological model computing unit simulates functions of the biological organs.

[Parameter set generating section : First embodiment]

[Step S1-1: inputting OGTT time-series data]

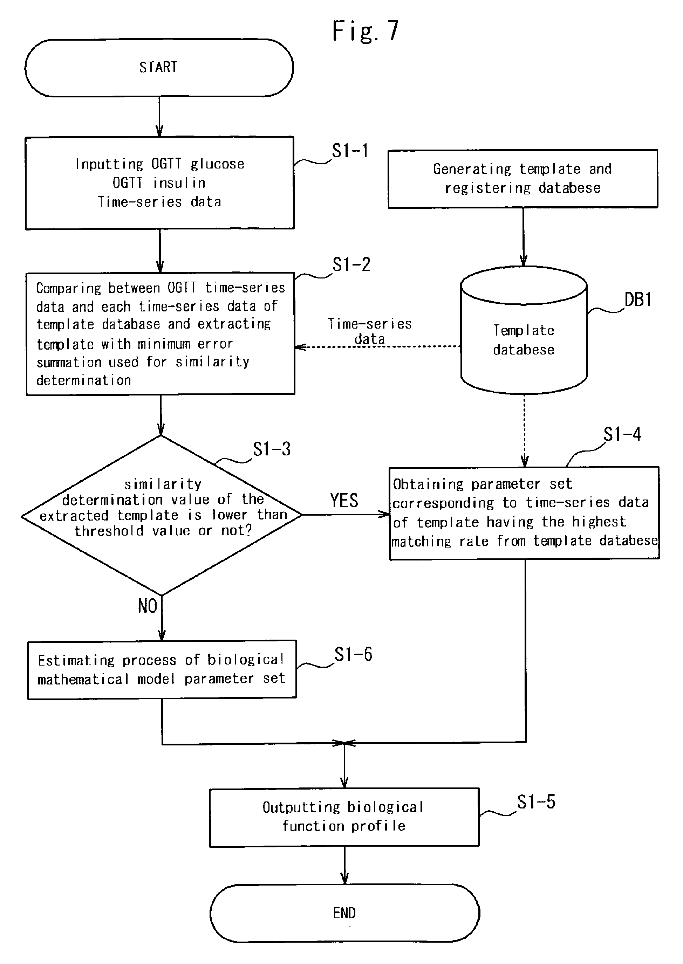

FIG. 7 is a flowchart showing procedures in which the parameter set generating section related to a first embodiment obtains a parameter set of the biological model. As shown in the flowchart, the procedure of obtaining parameters comprises a step of inputting OGTT (oral Glucose Tolerance Test) time-series data (Step S1-1).

OGTT time-series data are a result of OGTT (given amount of glucose solution is orally loaded to measure the time-series of blood glucose level and blood insulin concentration) from the actual examination of patients simulated by a biological model. The present system receives input as an actual biological response (actual examination values). Here, two data of and OGTT glucose data (blood glucose change data) and OGTT insulin (blood insulin concentration change data) are input as OGTT time-series data.

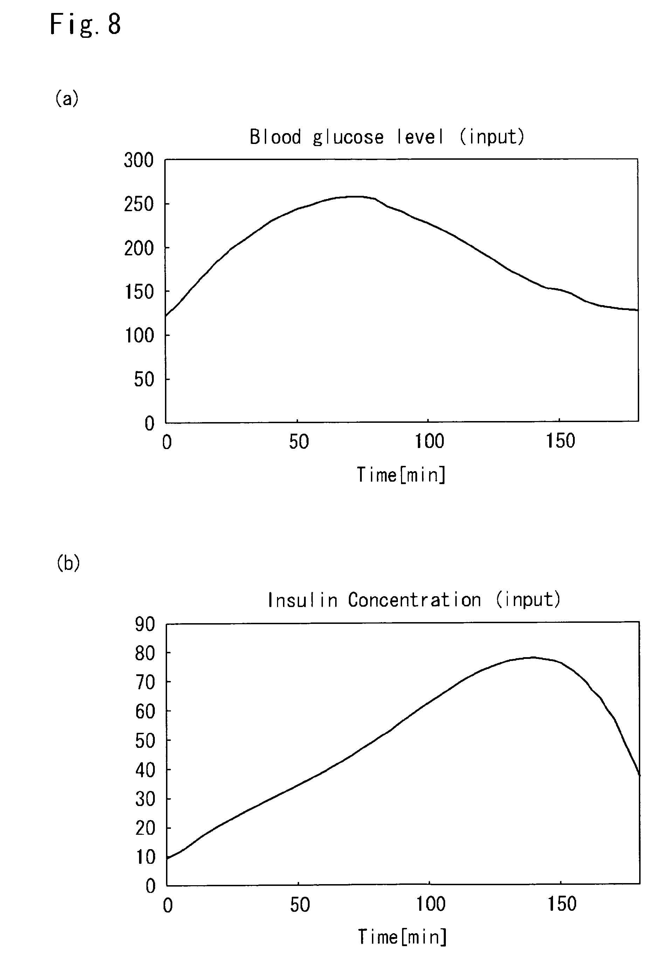

FIG. 8 shows the blood glucose level change data (FIG. 8(a)) and the blood insulin concentration change data (FIG. 8 (b)) as OGTT time-series data to be input.

In FIG. 8(a), the blood glucose level change data is measured data corresponding to time-series change of blood glucose level BG (t), one of output items in the biological model shown in FIGs. 2 to 6.

In FIG. 8 (b), the blood insulin concentration change data is measured data corresponding to time-series change of blood insulin concentration $I_1(t)$, one of output items in the biological model shown in FIGs. 2 to 6.

For inputting the OGTT time-series data to the present system, an input device 130 such as a keyboard and a mouse may be used. Or external memory device such as a database previously registered with OGTT time-series data.

[Step S1-2: template matching]

Next, this system (CPU 100a) matches the input OGTT time-series data to the template of template database DB1.

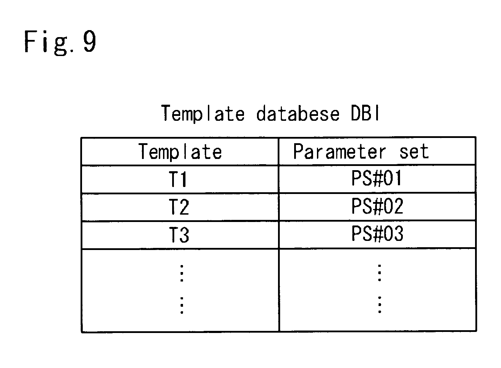

As shown in FIG. 9, the template database DB1 is preliminarily stored with a plurality sets of data, which are biological model reference output values T1, T2, ... as a template and parameter set PS#01, PS#02 ... correspondent to the reference output value to generate the reference output value. To make up a pair of reference output value and parameter set, a random reference output value is assigned by an appropriate parameter set, or on the contrary, a biological model output at the time when a random parameter set is selected is obtained by the biological simulation.

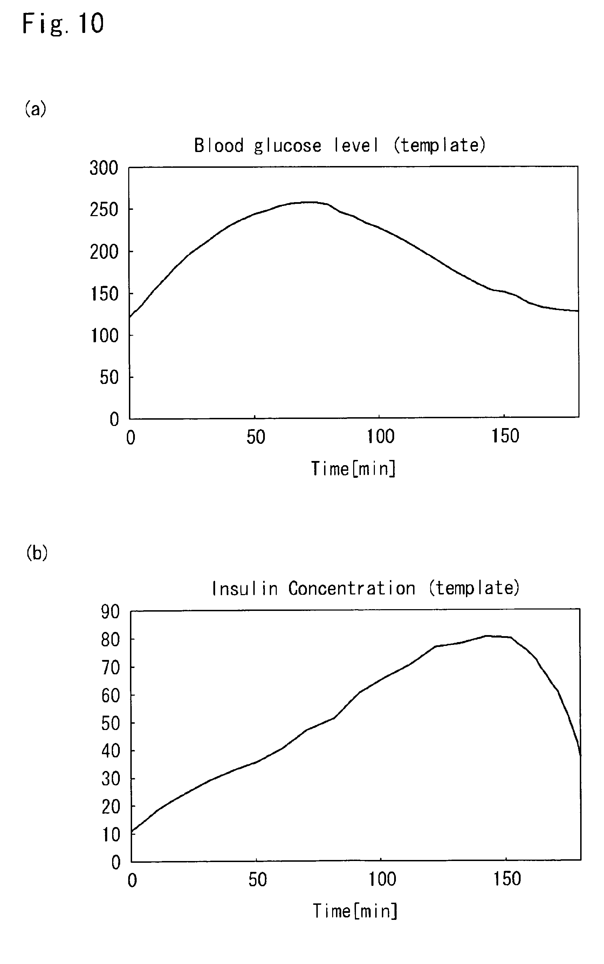

FIG. 10 shows an example of the template (reference output value) T1. FIG. 10(a) is a blood glucose change data as a template, which is reference time-series data corresponding to time-series change of the blood glucose level BG(t), one of output items in the biological model shown in FIGs. 2 to 6. FIG. 10(b) is blood insulin concentration change data as a template, which is reference time-series data corresponding to blood insulin concentration $I_1(t)$, one of output items in the biological model shown in FIGs. 2 to 6.

The system (CPU100a) computes similarity between each reference time-series datum of the above-mentioned template database DB1 and OGTT time-series data. The similarity is obtained by obtaining error summation. The error summation is obtained by the following formula.

$$
\begin{aligned}
\text{Error summation} = {} & \alpha \, \Sigma \, \big| BG(0) - BGt(0) \big| + \beta \, \Sigma \, \big| PI(0) - PIt(0) \big| \\
& + \alpha \, \Sigma \, \big| BG(1) - BGt(1) \big| + \beta \, \Sigma \, \big| PI(1) - PIt(1) \big| \\
& + \alpha \, \Sigma \, \big| BG(2) - BGt(2) \big| + \beta \, \Sigma \, \big| PI(2) - PIt(2) \big| \\
& + \ldots \\
= {} & \alpha \, \{ \, \Sigma \, \big| BG(t) - BGt(t) \big| \, \} + \beta \, \{ \, \Sigma \\
& \big| PI(t) - PIt(t) \big| \, \}
\end{aligned}
$$

where

BG: input data blood glucose level(mg/dl)
PI: input data blood insulin concentration [$\mu$U/ml]
BGt: template blood glucose level[mg/dl]
Pit: template blood insulin concentration [$\mu$U/ml]
t: time[minute]

Here, $\alpha$ and $\beta$ are coefficient used for normalization
$\alpha = 1/\text{Average} \{\Sigma BG(t)\}$
$\beta = 1/\text{Average} \{\Sigma PI(t)\}$
The average of the formula shows average level to all templates stored in the template database DB1.

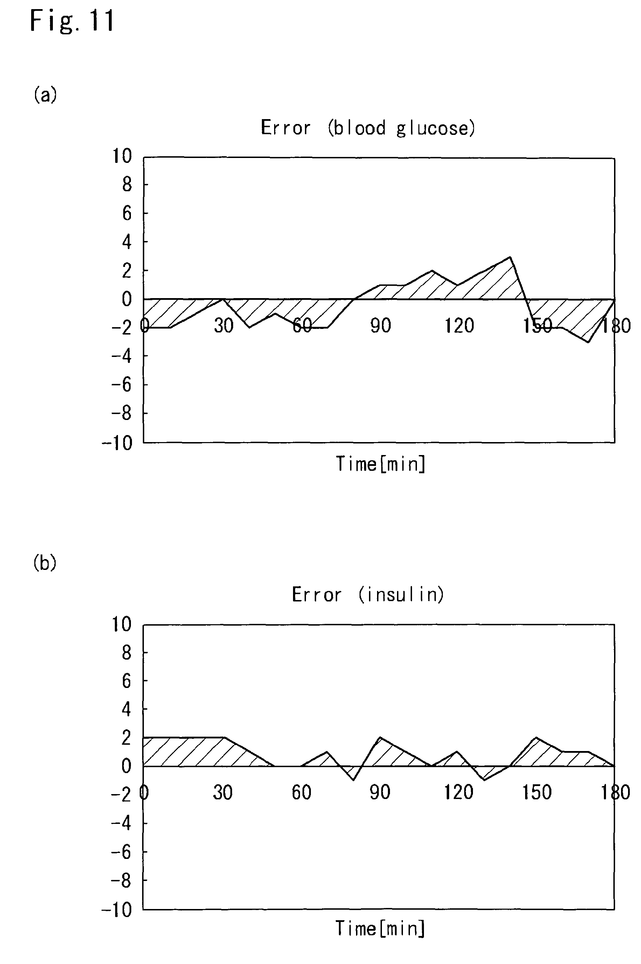

FIG. 11 shows the OGTT time-series error summation(no normalization) to the template T1. More specifically, FIG. 11(a) shows an error between the blood glucose level of FIG. 8(a) and the blood glucose level of FIG. 10(a). FIG. 11(b) shows an error between the insulin of FIG. 8(b) and the insulin of FIG. 10(b).
Based on FIG. 8 input data (date in the range of 0 to 180 minutes every 10 minutes) and FIG. 10 template T1,

$$
\Sigma \, \big| BG(t) - BGt(t) \big| = 29
$$

$$
\Sigma \, \big| PI(t) - PIt(t) \big| = 20
$$

where, provided $\alpha = 0.00035$, $\beta = 0.00105$

$$\text{error summation} = (0.00035 \text{ x } 29) + (0.00105 \text{ x } 20)$$
$$= 0.03115$$

Thus, CPU 100a obtains an error summation to each template in the template database DB1, and determines the template having the minimum error summation (similarity). Thus, CPU100a determines the template which is the most approximate to OGTT time-series data (Step S1-2).

[Step S1-3: Judging threshold value]

Consequently, in a step S1-3, CPU100a judges whether the template error summation (similarity) which has been determined in the step S1-2 is lower than a threshold value so as to judge whether the determined template is sufficiently similar to the input OGTT time-series data. For example, in the case that a threshold value (criterion) is set to 0.1 and that the above-mentioned template T1 (error summation =0.03115) is extracted in the Step S1-2, The template T1 is judged to be similar to the OGTT time-series data.

[Step S1-4: obtaining parameters]

Further, in a step S1-4, the CPU100a obtains from template database DB1 a parameter set corresponding to the template which has been determined in the step S1-2 and has been judged to be similar in the step S1-3. That means, a parameter set PS#01 corresponding to the template T1 is obtained (Ref. to FIG. 9).
This system selects a parameter set by the step S1-2, the step S1-4 using the template database DB1. This function constructs a means for referring database in this system.
Table 1 below exemplifies the specific numeral values of the parameter values included in the parameter set PS#01 obtained by the above-mentioned way.

Table. 1

| Parameter set PS#01 to Template T1 | | | |
|---|---|---|---|
| | Parameter | Value | Unit |
| Pancreas | h | 92. 43 | [mg/dl] |
| | $\alpha$ | 0.228 | [1/min] |
| | $\beta$ | 0.357 | $[(\mu U/ml) \cdot (dl/mg) \cdot (1/min)]$ |
| | M | 1 | [1/min] |
| | X(0) | 336. 4 | $[\mu U/ml]$ |
| | Y(0) | 4.4 | $[(\mu U/ml) \cdot (1/min)]$ |
| Insulin Kinetics | $A_1$ | 0.025 | [1/min] |
| | $A_2$ | 0.042 | [1/min] |
| | $A_3$ | 0.435 | [1/min] |
| | $A_4$ | 0.02 | [1/min] |
| | $A_5$ | 0.394 | [1/min] |
| | $A_6$ | 0.142 | [1/min] |
| Peripheral Metabolism | Kb | 0.009 | [1/min] |
| | Kp | 5.28E-05 | $[(ml/\mu U) \cdot (1/min)]$ |
| | u | 0.6 | |

(continued)

| Parameter set PS#01 to Template T1 | | | |
|---|---|---|---|
| | Parameter | Value | Unit |
| Hepatic Metabolism | $A_7$ | 0.47 | |
| | Kh | 0.0000462 | [(ml/$\mu$U) · (1/min) · (dl/kg)] |
| | b2 | 1.1 | |
| | r | 0.98 | |
| | $\alpha$2 | 0.228 | |
| | |4off | 5 | [$\mu$U/ml] |

The above-mentioned parameter set PS#01 is given to the biological model to generate the output approximate to the input OGTT time-series data, so that patients' biological organs can be appropriately simulated.

[Step S1-5: outputting biological function profiles]

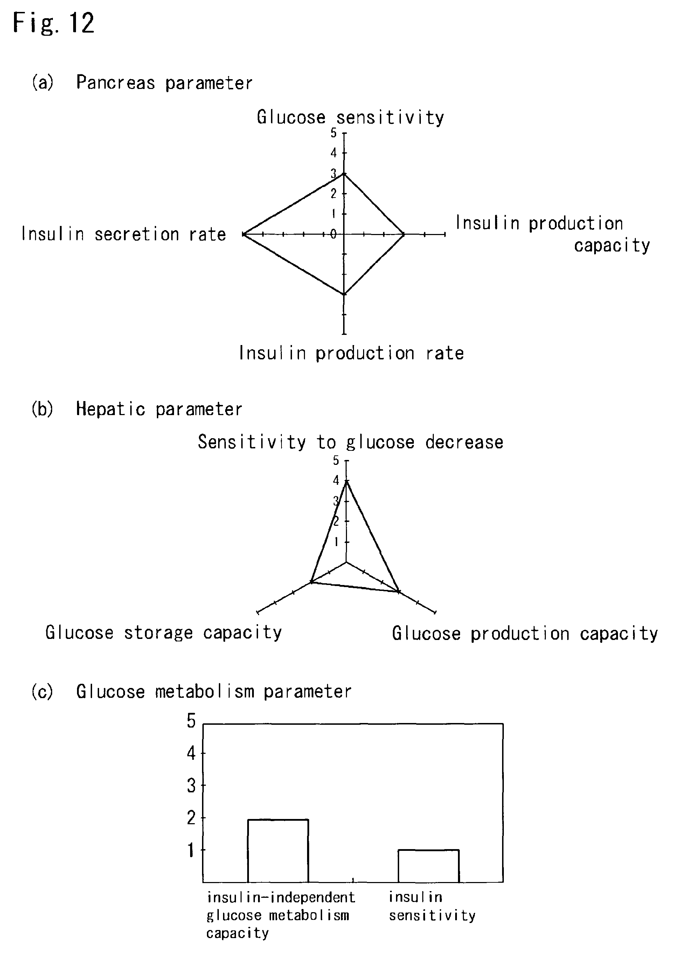

And then, this system (CPU100a) produces a biological function profile shown in FIG. 12 based on each parameter value included in the obtained parameter set PS#01 and outputs it on the display 120. FIG. 12 (a) is a pancreas profile which is produced based on the pancreas model block parameters, FIG. 12(b) is a hepatic metabolism profile based on the hepatic metabolism model block parameters, FIG. 12 (c) is a glucose metabolism profile based on the peripheral metabolism model block parameters.

[Step S1-6: estimating set biological model parameter]

In the step S1-3, when the error summation (similarity) of the template is judged to be higher than the threshold value (not similar), a parameter set is generated by the following parameter estimation process without using the template database DB1.

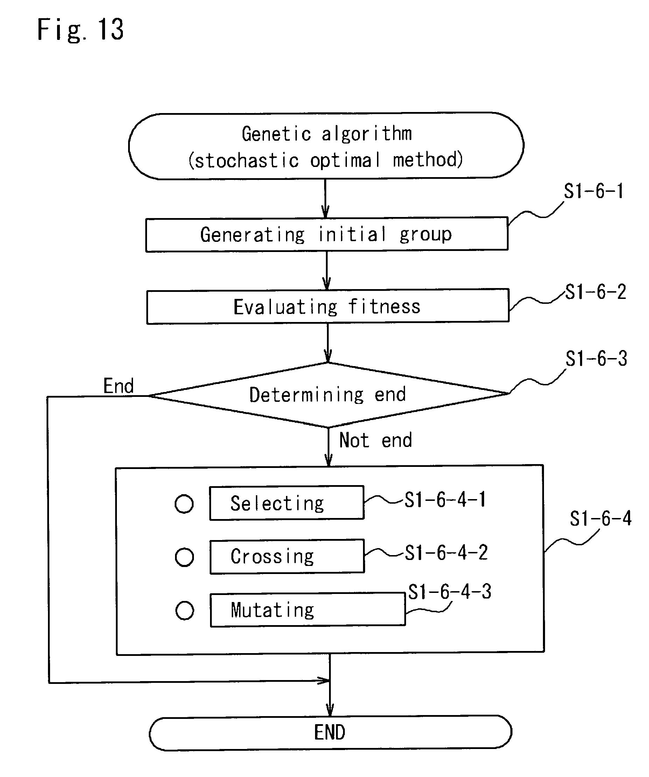

FIG. 13 is a flowchart of procedures of estimating parameters by genetic algorithm (simply referred to as "GA" hereinafter).
The procedure of generating the parameter set candidates by GA comprises, as shown in FIG. 13, a step of generating initial group of parameter set (Step S1-6-1), a step of evaluating fitness (Step S1-6-2), a step of selecting, crossing, and mutating (Step S1-6-4), and a step of determining end (Step S1-6-3, S1-6-5). These steps are executed by the CPU100a.
The algorithm in FIG. 13 will be described in detail hereinafter.

[Step S1-6-1: generating initial group]

This system has search-range information for each biological model parameter as shown in the following table 2. The search-range of the table 2 is the range which human being can take per parameter and the search-range of the table 2 is referred to as basic search range.
The system has functions to generate random numbers per parameter within a range of maximum value and minimum value of the table 2. thereby automatically and randomly generating parameter set PS. The parameter set PS obtained in this way may be referred to as "individual".

Table 2

| | Parameter | Minimum value | Maximum value | Unit |
|---|---|---|---|---|
| | | Parameter | Minimum value | Maximum value | Unit |
| **Default parameter search range** | | | | |
| Pancreas | h | 21.06 | 526.5 | [mg/dl] |
| | $\alpha$ | 0.00304 | 0. 684 | [1/min] |
| | $\beta$ | 0.0751168 | 338.0256 | $[(\mu U/ml) \cdot (dl/mg) \cdot (1/min)]$ |
| | M | 0.02 | 1 | [1/min] |
| | X (0) | 67.28 | 15138 | $[\mu U/ml]$ |
| | Y (0) | 0.88 | 198 | $[(\mu U/ml) \cdot (1/min)]$ |
| Insulin Kinetics | $A_1$ | 0.005 | 0.075 | [1/min] |
| | $A_2$ | 0.0084 | 0.126 | [1/min] |
| | $A_3$ | 0.087 | 1.305 | [1/min] |
| | $A_4$ | 0.004 | 0.06 | [1/min] |
| | $A_5$ | 0.0788 | 1.182 | [1/min] |
| | $A_6$ | 0.0284 | 0.426 | [1/min] |
| Peripheral Metabolism | Kb | 0.0018 | 0.027 | [1/min] |
| | Kp | 6.66667E-07 | 0.001 | $[(ml/\mu U) \cdot (1/min)]$ |
| | u | 0.12 | 1. 8 | |
| Hepatic Metabolism | $A_7$ | 0.094 | 1.41 | |
| | Kh | 0.00000924 | 0.0001386 | $[(ml/\mu U) \cdot (1/min) \cdot (dl/kg)]$ |
| | b1 | 0.18 | 2.7 | |
| | b2 | 0.22 | 3. 3 | |
| | r | 0.196 | 1 | |
| | $\alpha 2$ | 0.00304 | 0.684 | |
| | $I_{4off}$ | 1 | 15 | $[\mu U/ml]$ |

An initial group consisting of multiple parameter sets PS (e.g. 10) is generated by repeating the step of generating random numbers every parameters within the search range of Table 2.

[Step S1-6-2: evaluating fitness]

This system performs fitness evaluation on generated individuals to select and extract some individual PS from individuals PS of the (initial) group.

In the fitness evaluation, observed OGTT time-series data (Ref. to FIGs. 8(a), 8(b)) which have been input in the step S1-1 of FIG. 7 are used as a reference. The actually measured data (biological response) used as a reference are the data which this system desires to reproduce as output of the biological model. If the same response with the reference is obtained even in the biological model which is applied with the generated parameter set, it is considered that the individual's fitness for the actually measured value is high.

Then, in the fitness evaluation of the generated parameter set, judged is similarity (fitness rate) between output (blood glucose data, blood insulin concentration date) of the biological model which is applied with the generated parameter set generated parameter set and the reference (OGTT glucose data, OGTT insulin data).

[Step S1-6-4-1: selecting]

Next, in this system, some individuals (for example, 4 individuals) are selected from a (initial) group based on the predetermined reference e.g. fitness rate, and designated as "parents". As for a selection reference, not only "parents" with high fitness rate but also some "parents" with low fitness rate may be included in expectation that the fitness rate will increase in "children" the later generation.

[Step S1-6-4-2: crossing]

Against the group of individuals selected as "parents" in the above selecting step, this system generates new two individuals as "children" by the following procedure.

First, (1) two individuals are selected at random from the selected group of individuals. Next, (2) Frequency of crossing with individuals each other is obtained (the number of parameters as subject to be exchanged). The crossing frequency is obtained by the following formula if a crossing probability is expressed by XR (a range of 0 to 1 range):

Crossing frequency = [XR x (the number of parameters held by one individual)]

[] is a gauss mark (e.g. [3,14]=3).

And then, (3) a crossing point is obtained. The crossing point is obtained by randomly generating integral values from 1 to parameter number (22 in the case of Table 2) at "crossing frequency". Finally, (4) new individuals are generated. Particularly, between 2 individuals selected in the step (1), parameters of the crossing points obtained in the step (3) are exchanged to generate new two individuals.

Repetition of the above steps (1) to (4) generates new individuals "children" (6 individuals in the above example) by an increment of individuals decreased by selection and a new group is generated

[Step S1-6-4-3: mutating]

Against all individuals of the new group, this system changes parameters of each individual with mutation probability MR (in the range of 0 to 1) by the following procedures.

For example, in a mutation process conducted on a given parameter of a given individual, random number R is generated in the range of 0 to 1. With $R \leqq MR$, the random number is generated within the search range shown in Table 2 and substituted for an original value. The same process is conducted on all parameters of all individuals.

[Step S1-6-3, S1-6-5: determining end condition]

Steps S1-6-2 to S1-6-4 are repeated as shown in FIG. 13. When individual having the highest fitness rate exists in the present group as a result of fitness rate evaluation in the step S1-6-2, GA process is terminated and the individual having the highest fitness rate in the group is regarded as a result of estimation (step S1-6-3).

When frequency of repetition steps S1-6-2 to S1-6-4 (fitness rate evaluation to mutation) exceeds predetermined frequency, the GA procedure is terminated and the individual (parameter set) having the highest fitness rate in the group is the estimation result (step S1-6-5). As a determination condition of end, the repetition frequency may be e.g. 300 times.

When the parameters obtained by the biological model parameter set estimation process as mentioned above is provided to the biological model, output approximate to input OGTT time-series data can be generated, so that it is possible to appropriately simulate patients' biological organs. Further, this system draft biological function profiles based on the parameter value included in the parameter sets which are obtained by the estimation process and output them to the display 120 (Step S1 -5).

According to the first embodiment, if one approximate to the OGTT time-series data exists in the template of the template database DB1, the parameter sets can be easily obtained with reference to said database DB1, so that processing speed is increased comparing with the case of generating parameter sets by a biological model parameter set estimation process alone.

When the template database DB1 has sufficient templates, a function for the biological model parameter set estimation process (S1-6) may be omitted.

[Parameter generating section: second embodiment]

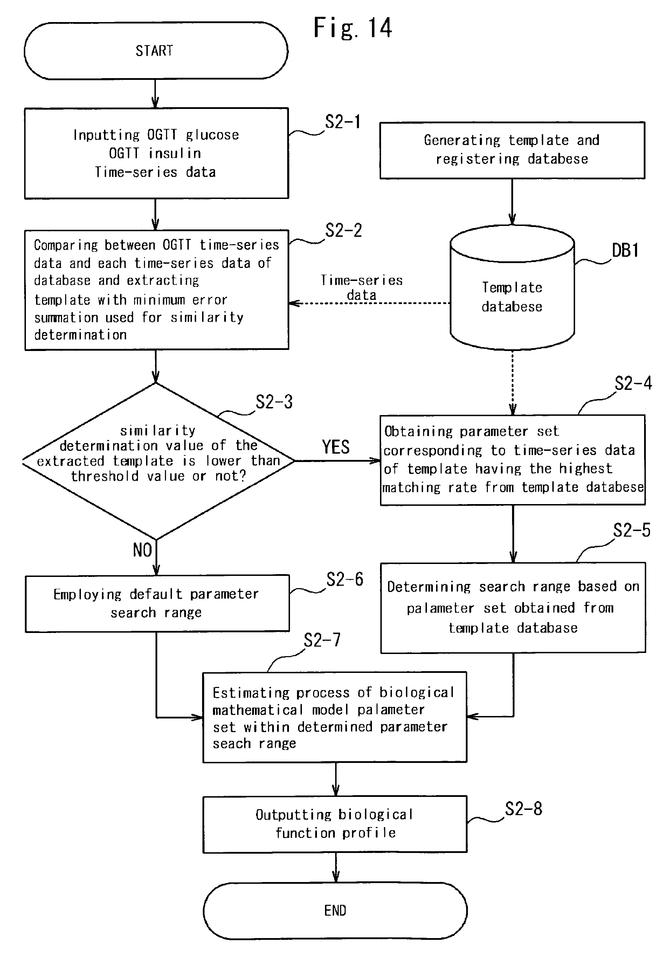

FIG. 14 shows a procedure by which a parameter set generating section related to the second embodiment obtains the biological model parameter sets. Step S2-1, Step S2-2, Step S2-3, Step S2-4, in FIG. 14 are respectively the same procedures with Step S1-1, Step S1-2, Step S1-3, Step S1-4 in FIG. 7.

In the second embodiment, as the result of template matching, in Step S2-4, the parameter set which has been obtained from the template database DB1 is not used as it is but CPU100a determines a local parameter search range based on said parameter set (Step S2-5).

The local search range determined in Step S2-5 includes each parameter value of the parameter set obtained in Step S2-4 and is narrower than the basic search range shown in the above Table 2. More specifically, the local search range has a predetermined search width of mm1 to mm22 with a parameter value of the parameter set obtained in Step S2-4 as a center value. That means, as shown in Table 3, each parameter value is set with search width of mm1 to mm22. For example, with regard to a parameter value "h" of the parameter set obtained in Step S2-4, "h - mm1" becomes the minimum value of the local search range of "h", "h + mm1" becomes the maximum value of the local search range.

Table 3

| Parameter search width | | |
|---|---|---|
| | Parameter | Search width |
| Pancreas | h | mm1 |
| | $\alpha$ | mm2 |
| | $\beta$ | mm3 |
| | M | mm4 |
| | X(0) | mm5 |
| | Y(0) | mm6 |
| Insulin Kinetics | $A_1$ | mm7 |
| | $A_2$ | mm8 |
| | $A_3$ | mm9 |
| | $A_4$ | mm10 |
| | $A_5$ | mm11 |
| | $A_6$ | mm12 |
| Peripheral Metabolism | Kb | mm13 |
| | Kp | mm14 |
| | u | mm15 |
| Hepatic Metabolism | $A_7$ | mm16 |
| | Kh | mm17 |
| | b1 | mm18 |
| | b2 | mm19 |
| | r | mm20 |
| | $\alpha2$ | mm21 |
| | |4off | mm22 |

While in Step S2-3, when the CPU 100a judges that the template error summation (similarity) is larger than the threshold value (dissimilarity), the default value parameter search range (basic parameter search range) shown in Table 2 is employed as a search range.

And in Step S2-7, the biological model parameter set estimation process is performed in the local research range determined in Step S2-5 or in the basic research range in Step S2-6. That means, parameters are generated by genetic algorithm for individual generation/crossing/mutating with the local search range or the basic search range. The procedure of genetic algorithm is the same with the procedure in Step S1-6 in FIG. 7.

The biological function profiles are generated based on the parameter sets generated in Step S2-7 and are output to the display 120 (Step S2-8).

Thus, the parameter set generating section of the second embodiment dose not use the parameter set obtained from the template database DB1 as it is, but determines a search range based on parameter sets obtained and search parameter sets generating output more approximate to the input OGTT time-series data with the search range, so that more appropriate parameter sets can be obtained.

In addition, in the second embodiment, the local search range narrower than the basic search range, is set based on the parameter set obtained from the template database DB1, so that process speed can be increased comparing with that parameter sets are generated by the biological model parameter set estimation process with the basic search range.

In the second embodiment, when template database DB1 has sufficient templates, the function of biological model parameter set estimation process with the basic search range may be omitted.

[Parameter set generating section: third embodiment]

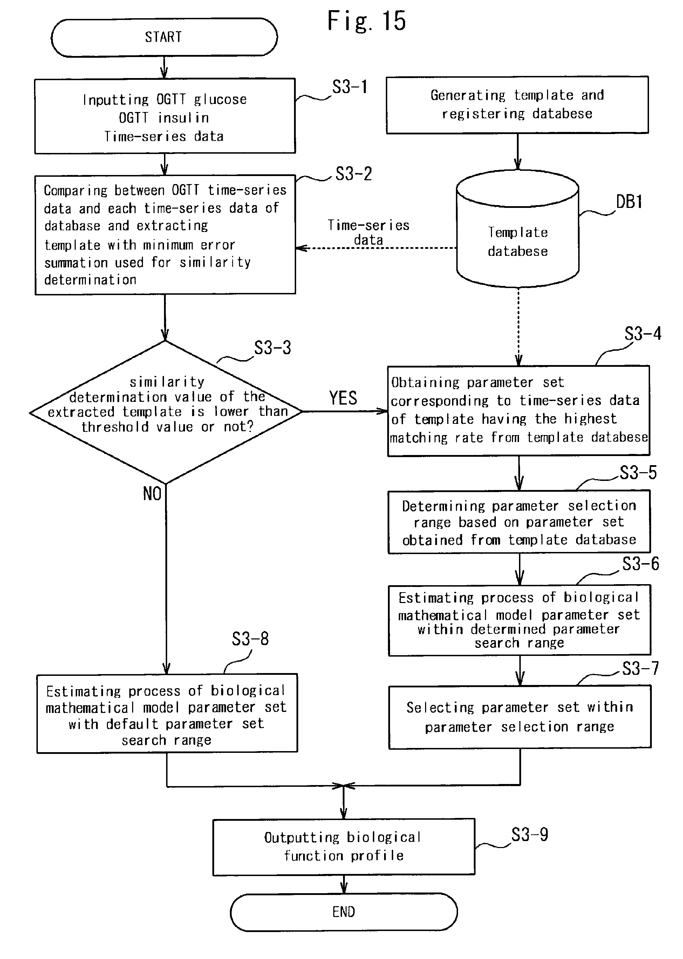

FIG. 15 shows a procedure by which a parameter set generating section related to the third embodiment obtains the biological model parameter sets. Step S3-1, Step S3-2, Step S3-3, Step S3-4, in FIG. 15 are respectively the same procedures with Step S1-1 (Step 2-1 of FIG. 14), Step S1-2 (Step S2-2 of FIG. 14), Step S1-3(Step 2-3 of FIG.14), Step S1-4(Step S2-4 of FIG.14) in FIG. 7.

Further, Step S3-5 in FIG. 15 is the same procedure with Step S2-5 in FIG. 14. However, in Step S2-5 in FIG. 14, "search range" is determined based on the parameter set obtained from the template database DB1, while Step S3-5 of the third embodiment, "selection range" is determined by the same process with Step S2-5.

This function of StepS3-5 comprises a selection range determining means in this system.

Step S3-6 of the third embodiment is not related with said "selection range" , but the biological model parameter set estimation process is performed with the default parameter search range (basic search range). That means, parameter sets are automatically generated with the basic search by generating individual/crossing/mutating genetic algorithm. The procedure of the genetic algorithm is the same with that of Step S1-6 in FIG. 7.

The parameter sets automatically generated during operation of the genetic algorithm are narrowed down in the fitness evaluation of the first selection and the parameter set approximate input ODTT time-series data is selected. The function of Step S3 to 6 comprises the first selecting means of this system.

Further, here, the genetic algorithm is executed several time to generate a plurality of parameter sets. When plurality of the generated parameter sets are provided to the biological model, each parameter set can generate output approximate to the input OGTT time-series data and there may be a plurality of parameter sets generating similar output. For example, parameter sets whose combination of parameter set values is impossible for human beings may be included in plurality of the parameter sets.

Then, in the third embodiment, a second selecting process is performed to narrow down plurality of the parameter sets obtained in the biological model parameter set estimation process in Step S3-6 with said "selection range" (Step S3-7). Because "selection" is a possible range where appropriate parameter values can exist, appropriate parameter sets cab be selected by selecting the parameter sets within "selection range" among a plurality parameter sets. Function of Step S-7 comprises a second selecting means of the this system.

Further, in the third embodiment, in Step S3-3, when the template error summation (similarity) is determined larger than the threshold value (non-similar), a single parameter set is generated by the biological model parameter set estimation process with default parameter search range (basic parameter search range) as a search range.

And the biological function profiles are generated based on the parameter set generated in Step S3-7 or the parameter set generated in Step S3-8 and are output to the display 120 (Step S3-9).

In the third embodiment, when template database DB1 has sufficient templates, the function of Step S3-8 may be omitted.

[Parameter set generating section: fourth embodiment]

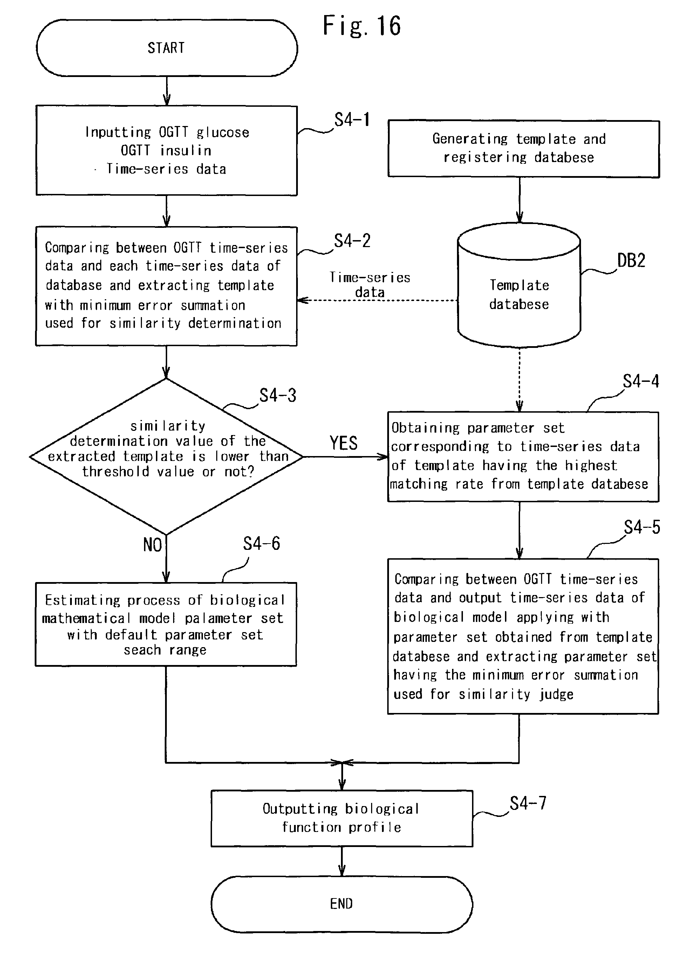

FIG. 16 shows a procedure by which a parameter set generating section related to the fourth embodiment obtains the biological model parameter sets. Step S4-1, Step S4-2, Step S4-3 in FIG. 16 are respectively the same procedures with Step S1-1, Step S1-2, Step S1-3 in FIG. 7.

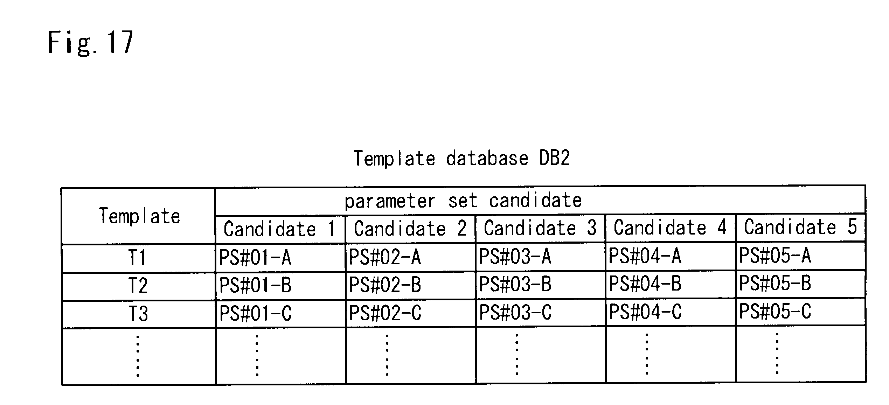

Further, Step S4-4 in FIG. 16 is the same procedure with Step S1-4 in FIG. 7. However, in Step S4-4, the template database DB2 shown in FIG. 17 is referred instead of the template data base DB1. The template database DB2 in FIG. 17 corresponds to the template (output for reference) and is assigned with a plurality of parameter set candidates. For example, a template T1 is assigned with five parameter set candidates PS#01-A, PS#02-A, PS#03-A, PS#04-A, PS#05-A. These five parameter set candidates has different parameter value with each other, but when they are provided to the biological model as a parameter, the biological model generates output approximate to the template T1.

In the fourth embodiment, when the template T1 is selected as a template most approximate to the OGTT time-series data, the template database DB2 is referred to obtain five parameter sets candidates PS#01-A to PS#05-A which correspond to the template T1 in Step S4-4.

When these candidates PS#01-A to PS#05-A are provided to the biological model, each of the candidates can generate output relatively approximate to OGTT time-series data (template T1). However their output are slightly different from each other.

And, in Step S4-5, the CPU100a similarity computation (error summation computation) same as template matching is performed on OGTT time-series data and output of the biological model provided with each of the parameter set candidates PS#01-A to PS#05-A. The parameter candidate of the minimum error summation is available to generate output most approximate to the OGTT time series data.

In the template database DB2, the number of parameter set candidates corresponding to one template is not limited to five. Any number may be possible.

Further in the fourth embodiment, in StepS4-3, when the template error summation(similarity) is determined larger than the threshold value(non-similar), a parameter set is generated by the biological model parameter set estimation process with default parameter search range(basic parameter search range) as a search range.

And biological function profiles are generated based on the parameter set obtained in StepS4-5 or Step S4-6, and they are output to the display 120(Step S4-7).

In the forth embodiment, when template database DB1 has sufficient templates, the function of Step S3-7 may be omitted.

[Parameter set generating section: fifth embodiment]

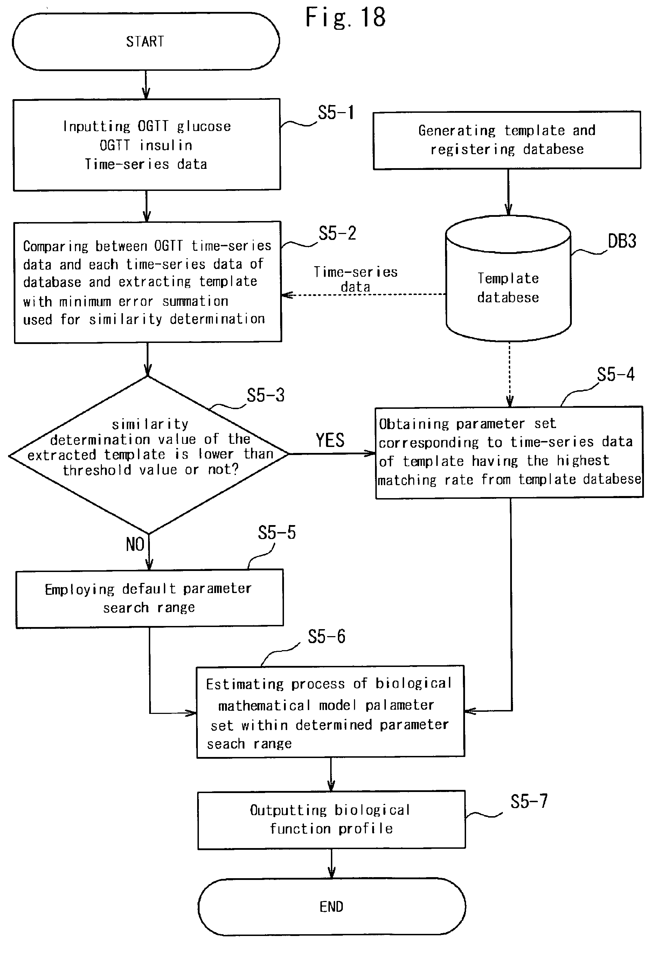

FIG. 18 shows a procedure by which a parameter set generating section related to the fifth embodiment obtains the biological model parameter sets. Step S5-1, Step S5-2, Step S5-3, in FIG. 18 are respectively the same procedures with Step S1-1, Step S1-2, Step S1-3 in FIG. 7.

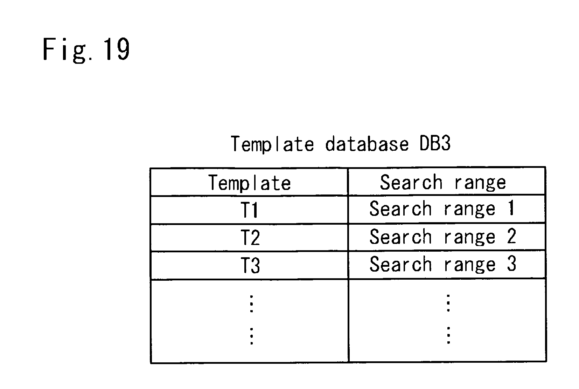

Although Step S1-4 in FIG. 18 is almost same procedures with Step S5-4 of FIG. 7 and Step S2-4 of FIG. 14, referred is not the template database DB1 but the template database DB3 shown in Step S5-4 in FIG. 19. The template database D3 to FIG. 19 is assigned with the search range of parameter corresponding to a single template (output for reference). For example, the ranges shown in the following table 4 are set as a search range corresponding to the template T1.

Table 4

| Search Range 1 corresponding to template T1 | | | | |
|---|---|---|---|---|
| | Parameter | Minimum value | Maximum value | Unit |
| Pancreas | h | 105.3 | 175.5 | [mg/dl] |
| | $\alpha$ | 0.0152 | 0.228 | [1/min] |
| | $\beta$ | 0.0751168 | 112.6752 | [($\mu$U/ml) · (dl/mg) · (1/min)] |
| | M | 0.1 | 1 | [1/min] |
| | X(0) | 336.4 | 5046 | [$\mu$U/ml] |
| | Y(0) | 4.4 | 66 | [($\mu$U/ml) · (1/min)] |
| Insulin Kinetics | $A_1$ | 0.025 | 0.025 | [1/min] |
| | $A_2$ | 0.042 | 0.042 | [1/min] |
| | $A_3$ | 0.435 | 0.435 | [1/min] |
| | $A_4$ | 0.02 | 0.02 | [1/min] |
| | $A_5$ | 0.394 | 0.394 | [1/min] |
| | $A_6$ | 0.142 | 0.142 | [1/min] |
| Peripheral Metabolism | Kb | 0.009 | 0.009 | [1/min] |
| | Kp | 3.33333E-06 | 0.00033333 | [(ml/$\mu$U) · (1/min)] |
| | u | 0.6 | 0.6 | |
| | $A_7$ | 0.47 | 0.47 | |
| | Kh | 0.0000462 | 0.0000462 | [(ml/$\mu$U) · (1/min) · (dl/kg)] |
| Hepatic Metabolism | b1 | 0.9 | 0.9 | |
| | b2 | 1.1 | 1.1 | |
| | r | 0.98 | 0. 98 | |
| | $\alpha$2 | 0.0152 | 0.228 | |
| | I4off | 5 | 5 | [$\mu$U/ml] |

In the template database DB3, the search range corresponding to the template is narrower than the default parameter search range (basic search range) and the random parameter set in said search range is provided to the biological model to generate output approximate to the template. That means, the search range of Table 4 is same with the previously -mentioned local search range.

In this fifth embodiment, as in the second embodiment, the search range narrower than the basic search range can be determined based on the template and the local search range is stored in the template database DB3, so that the local search range is immediately obtained and a processing speed is increased.

In Step S5-3, when the template error summation (similarity) is determined larger than the threshold value (non-similar), the default parameter search value range (basic parameter search range) is employed as a search range (Step S5-5). biological model parameter set estimation process is performed with the local search range determined in Step S5-4, or the basic search range determined in Step S5-5. That means, parameter sets are generated with the local search range or the basic search range by genetic algorithm performing generating individuals/crossing/mutating. Te procedure of the genetic algorithm is same with the procedure in Step S1-6 in FIG.7.

As mentioned above, the function of Step S5-6 comprises a selecting means for selecting parameters in this system.

The biological function profiles are generated based on the parameter set generated in Step S5-6 and they are output to the display 120 (Step S5-7).

[Parameter set generating section: sixth embodiment]

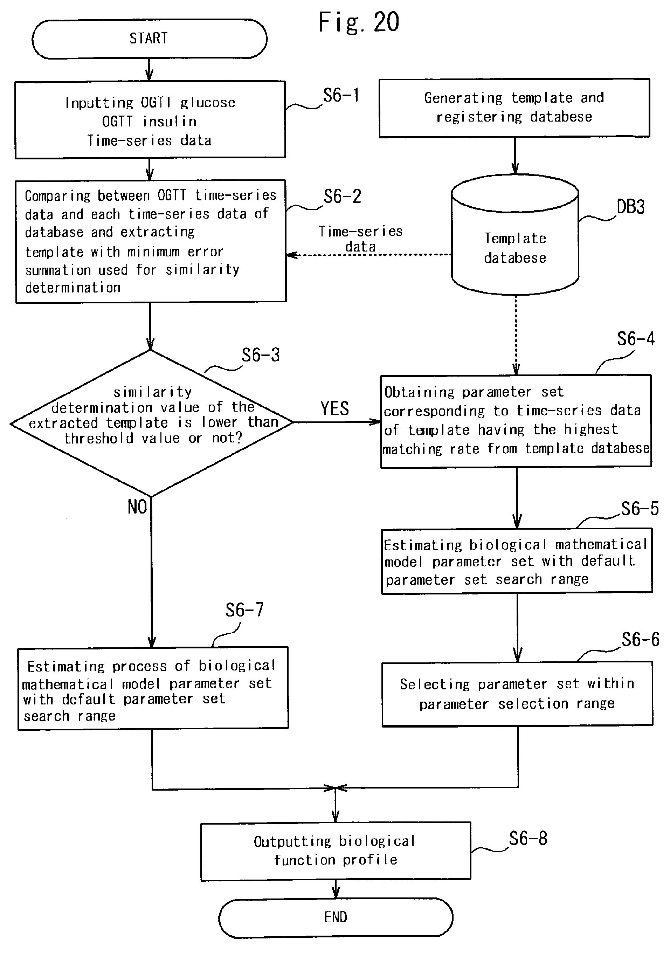

FIG. 20 shows a procedure by which a parameter set generating section obtains the biological models related to the sixth embodiment obtains the parameter sets of biological model. Step S6-1, Step S6-2, Step S6-3 in FIG. 20 are respectively the same procedures with Step S1-1, Step S1-2, Step S1-3 in FIG. 7.

Step S6-4 in FIG. 20 is same with Step S5-4 in FIG. 18.

However, the parameter set "search range" is obtained from the template database DB3 in Step S5-4 in FIG. 18, while parameter set "selection range" is obtained in Step S6-4 of the sixth embodiment as in Step S5-4.

Further, in Step S6-5, as in Step S3-6 of the third embodiment, the biological model parameter set estimation process is performed with the default parameter search range (basic search range) without regard to said "selection range". That means, parameter sets are automatically generated with the basic search range by the genetic algorithm for generating individual/crossing/mutating. The procedure of the genetic algorithm is same with Step S1-6 in FIG. 7.

The parameter sets automatically generated during the genetic algorithm execution are narrowed down by the first selection with fitness evaluation in the genetic algorithm and the parameter set approximate to the input OGTT time-series data is selected.

Here, the genetic algorithm is executed several times to generate a plurality of parameter sets.

Thus, the function of Step S6-5 comprises the first selecting means of this system.

Then, in the sixth embodiment, as in Step S3-7 of the third embodiment, a second selecting process is performed to narrow down plurality of the parameter sets obtained in the biological model parameter set estimation process with said "selection range" (Step S6-6). Because "selection range" is a available range where appropriate parameter values exist, appropriate parameter sets cab be selected by selecting the parameter sets within "selection range" among a plurality parameter sets. Function of Step S-6-6 comprises a second selecting means of the this system.

Further, in the sixth embodiment, in Step S6-3, when the template error summation (similarity) is determined larger than the threshold value (dissimilarity), a single parameter set is generated by the biological model parameter set estimation process with default parameter search range (basic parameter search range) as a search range (Step S6-7).

And the biological function profiles are generated based on the parameter set generated in Step S6-6 or the parameter set generated in Step S6-7 and are output to the display 120 (Step S6-8).

In the sixth embodiment, when template database DB1 has sufficient templates, the function of Step S6-7 maybe omitted.

The present invention is not to be restricted to the above mentioned embodiments, and various modification may be possible without departing from the spirit and scope of the invention.

For example, a subject to be simulated is not limited to diabetes pathological conditions, but may be other pathological conditions. And constructions of biological model and its parameters are not limited to the above mentioned ones and may be changed accordingly.

Further, a searching means(selecting means) is not limited to one comprising the genetic algorithm. Other algorithm is satisfied as long as parameter sets are randomly and automatically generated and appropriate parameter sets are selected with appropriate reference.

**Claims**

1. A biological simulation system using a biological model comprising:

an internal parameter set generating section generating internal parameter sets constituting a biological model;
a biological model computing section computing output of a biological model which emulates a biological response of a biological organ based on the internal parameter set; and
a template database having a plurality of combinations of a reference output value of the biological model and an internal parameter set corresponding to the reference output value,

wherein said internal parameter set generating section comprises a database reference means which selects a reference output value approximate to an actual biological response from said template database and which selects an internal parameter set corresponding to the selected reference output value.

2. The biological simulation system according to Claim 1, wherein said internal parameter set generating section further comprises a parameter set searching means for searching internal parameter set which generates output more approximate to the actual biological response based on the internal parameter set which has been selected by said database reference means.

3. The biological simulation system according to Claim 2, wherein said internal parameter set generating section comprises a search range determining means for determining a search range of the internal parameter set based on the internal parameter set which has been selected by said database reference means, said parameter set searching means being constructed so as to search said parameter set within the search range which has been selected by set said search range determining means.

4. The biological simulation system according to Claim 2
wherein said internal parameter set generating section further comprises a selection range determining means for determining a selection range of internal parameter set based on the internal parameter set which has been selected by said database reference means, and said parameter set searching means comprises: a means for automatically generating a plurality of different internal parameter sets; a first selecting means which determines an approximation between biological model output calculated applying the internal parameter set automatically generated and an actual biological response corresponding to said output and which selects an appropriate internal parameter sets from a plurality of the internal parameter sets; and a second selecting means for selecting parameter sets within said selection range out of the internal parameter sets which has been obtained by said first selecting means.

5. A biological simulation system using a biological model comprising:

an internal parameter set generating section generating internal parameter sets constituting a biological model;
a biological model computing section computing output of a biological model which emulates a biological response of a biological organ based on the internal parameter set; and
a template database having a plurality of combinations of a reference output value of the biological model and a search range of an internal parameter set corresponding to the reference output value;

wherein said internal parameter set generating section comprises:

a database reference means which selects a reference output value approximate to an actual biological response from said template database and which selects a search range of an internal parameter set corresponding to the selected reference output value;
a means for automatically generating a plurality of different internal parameter sets within said search range; and
a selecting means which determines an approximation between biological model output calculated applying the internal parameter set automatically generated and an actual biological response corresponding to said output and which selects an appropriate internal parameter set from a plurality of the internal parameter sets generated.

6. A biological simulation system using a biological model comprising:

an internal parameter set generating section generating internal parameter sets constituting a biological model;
a biological model computing section computing output of a biological model which emulates a biological response of a biological organ based on the internal parameter set; and

a template database having a plurality of combinations of a reference output value of the biological model and an internal parameter set selection range corresponding to the reference output value,

wherein said internal parameter set generating section comprises:

a database reference means which selects a reference output value approximate to an actual biological response from said template database and which selects a selection range of an internal parameter set corresponding to the selected reference output value;
a means for automatically generating a plurality of different internal parameter sets;
a first selecting means which determines an approximation between biological model output calculated applying the internal parameter set automatically generated and an actual biological response corresponding to said output and which selects an appropriate internal parameter set from a plurality of the internal parameter sets automatically generated; and
a second selecting means for selecting parameter sets within said selection range out of the internal parameter sets which has been obtained by said first selecting means.

7.  A computer program product stored in a computer-readable medium for processing a biological simulation which is executed by a computer, comprising:

a program code for generating internal parameter sets constituting a biological model, and
a program code for computing output of the biological model emulating a biological response of a biological organ based on the internal parameter set,

wherein said program code for generating said internal parameter sets comprises a program code which selects reference output values approximate to the actual biological response from a template database having a plurality of combinations of reference output value of the biological model and the internal parameter set corresponding to said reference output value and which selects internal parameter sets corresponding to the selected reference output values.

Fig. 1

100
(100a)

Simulation System

Main Body

110

110a CPU

110c RAM

ROM 110b

110d Application Program 140a

Input/out Interface 110f

Readout Device 110e

140 140a

Image Output Interface 110h

110i

130 Input Device

120

Fig. 2

Fig. 3

Blood Glucose
Level BG

Insulin
Secretion
Rate SR

Fig. 4

## Fig. 5

Fig. 6

## Fig. 7

```
        START
          │
          ▼
┌──────────────────────┐  S1-1         ┌──────────────────────┐
│  Inputting OGTT glucose │             │  Generating template and │
│     OGTT insulin       │             │   registering databese   │
│   Time-series data     │             └──────────────────────┘
└──────────────────────┘                         │
          │                                        ▼
          ▼                                    ┌────────┐
┌──────────────────────┐  S1-2               │Template │  DB1
│ Comparing between OGTT time-series│        │database │
│ data and each time-series data of│◄┈┈┈┈┈┈┈└────────┘
│ template database and extracting │ Time-series
│ template with minimum error      │   data          │
│ summation used for similarity    │                 ┊
│ determination                    │                 ┊
└──────────────────────┘                             ┊
          │                                           ▼
          ▼                              ┌──────────────────────┐ S1-4
      ╱────────╲  S1-3                   │Obtaining parameter set │
     ╱ similarity ╲                      │corresponding to time-series data│
    ╱ determination value of the ╲  YES  │of template having the highest│
    ╲ extracted template is lower than╲─►│matching rate from template databese│
     ╲ threshold value or not? ╱        └──────────────────────┘
       ╲────────╱                                   │
          │ NO                                      │
          ▼                                         │
┌──────────────────────┐ S1-6                       │
│ Estimating process of biological│                 │
│ mathematical model parameter set│                 │
└──────────────────────┘                            │
          │                                          │
          └──────────────┬───────────────────────────┘
                         ▼
              ┌──────────────────────┐ S1-5
              │  Outputting biological │
              │    function profile    │
              └──────────────────────┘
                         │
                         ▼
                       END
```

26

# Fig. 8

(a)

Blood glucose level (input)

(b)

Insulin Concentration (input)

## Fig. 9

Template databese DBI

| Template | Parameter set |
|----------|---------------|
| T1 | PS#01 |
| T2 | PS#02 |
| T3 | PS#03 |
| ⋮ | ⋮ |

# Fig. 10

(a)

Blood glucose level (template)

(b)

Insulin Concentration (template)

# Fig. 11

(a)

Error (blood glucose)

(b)

Error (insulin)

# Fig. 12

(a)  Pancreas parameter

Glucose sensitivity

Insulin secretion rate

Insulin production capacity

Insulin production rate

(b)  Hepatic parameter

Sensitivity to glucose decrease

Glucose storage capacity

Glucose production capacity

(c)  Glucose metabolism parameter

insulin-independent glucose metabolism capacity

insulin sensitivity

Fig. 13

Genetic algorithm
(stochastic optimal method)

Generating initial group — S1-6-1

Evaluating fitness — S1-6-2

Determining end — S1-6-3

End

Not end

○ Selecting — S1-6-4-1

○ Crossing — S1-6-4-2

○ Mutating — S1-6-4-3

S1-6-4

END

# Fig. 14

# Fig. 15

```
┌─────────────────┐
│      START      │
└─────────────────┘
         │
         ▼
┌─────────────────────────┐  S3-1      ┌─────────────────────────┐
│  Inputting OGTT glucose │            │  Generating template and│
│       OGTT insulin      │            │   registering databese  │
│     Time-series data    │            └─────────────────────────┘
└─────────────────────────┘                        │
         │                                          ▼
         ▼                                       ┌──────┐
┌─────────────────────────┐  S3-2               │Template│  DB1
│ Comparing between OGTT  │        Time-series  │databese│
│ time-series data and    │◄ ─ ─ ─ data ─ ─ ─  └──────┘
│ each time-series data of│
│ database and extracting │
│ template with minimum   │
│ error summation used for│
│ similarity determination│
└─────────────────────────┘
```

**S3-3** — similarity determination value of the extracted template is lower than threshold value or not?

YES → **S3-4** Obtaining parameter set corresponding to time-series data of template having the highest matching rate from template databese

**S3-5** Determining parameter selection range based on parameter set obtained from template database

**S3-6** Estimating process of biological mathematical model parameter set within determined parameter search range

**S3-7** Selecting parameter set within parameter selection range

NO → **S3-8** Estimating process of biological mathematical model parameter set with default parameter set search range

**S3-9** Outputting biological function profile

```
┌─────────────────┐
│       END       │
└─────────────────┘
```

## Fig. 16

```
                    START
                      │
                      ▼
        ┌─────────────────────────┐
        │ Inputting OGTT glucose  │ ──── S4-1
        │   OGTT insulin          │
        │ · Time-series data      │
        └─────────────────────────┘
                      │
                      ▼
```

```
┌────────────────────────────────┐
│ Comparing between OGTT time-series │ ──── S4-2
│ data and each time-series data of  │
│ database and extracting template   │
│ with minimum error summation       │
│ used for similarity determination  │
└────────────────────────────────┘
```

```
┌─────────────────────────┐
│ Generating template and │
│   registering databese  │
└─────────────────────────┘
            │
            ▼
        Template
        databese ──── DB2
```

Time-series data

```
            similarity
     determination value of the     ── YES ──►
     extracted template is lower than
            threshold value or not?    S4-3
```

```
┌───────────────────────────────┐
│ Obtaining parameter set        │ ──── S4-4
│ corresponding to time-series data │
│ of template having the highest │
│ matching rate from template databese │
└───────────────────────────────┘
```

NO

```
┌───────────────────────────────┐
│ Estimating process of biological │ ──── S4-6
│ mathematical model palameter set │
│ with default parameter set      │
│ seach range                     │
└───────────────────────────────┘
```

```
┌───────────────────────────────┐
│ Comparing between OGTT time-series │ ──── S4-5
│ data and output time-series data of │
│ biological model applying with   │
│ parameter set obtained from template │
│ databese and extracting parameter set │
│ having the minimum error summation │
│ used for similarity judge        │
└───────────────────────────────┘
```

```
        ┌─────────────────────────┐
        │ Outputting biological   │ ──── S4-7
        │   function profile      │
        └─────────────────────────┘
                      │
                      ▼
                    END
```

Fig. 17

Template database DB2

| Template | parameter set candidate | | | | |
| --- | --- | --- | --- | --- | --- |
| | Candidate 1 | Candidate 2 | Candidate 3 | Candidate 4 | Candidate 5 |
| T1 | PS#01-A | PS#02-A | PS#03-A | PS#04-A | PS#05-A |
| T2 | PS#01-B | PS#02-B | PS#03-B | PS#04-B | PS#05-B |
| T3 | PS#01-C | PS#02-C | PS#03-C | PS#04-C | PS#05-C |
| ⋮ | ⋮ | ⋮ | ⋮ | ⋮ | ⋮ |

EP 1 722 345 A1

# Fig. 18

START

Inputting OGTT glucose
OGTT insulin
Time-series data    S5-1

Comparing between OGTT time-series
data and each time-series data of
database and extracting template
with minimum error summation
used for similarity determination    S5-2

similarity
determination value of the
extracted template is lower than
threshold value or not?    S5-3

Generating template and
registering databese

Time-series
data

Template
databese    DB3

YES

Obtaining parameter set
corresponding to time-series data
of template having the highest
matching rate from template databese    S5-4

NO

Employing default parameter
search range    S5-5

Estimating process of biological
mathematical model palameter
set within determined parameter
seach range    S5-6

Outputting biological
function profile    S5-7

END

Fig. 19

Template database DB3

| Template | Search range |
|----------|--------------|
| T1 | Search range 1 |
| T2 | Search range 2 |
| T3 | Search range 3 |
| ⋮ | ⋮ |

## Fig. 20

```
        ( START )
             |
             v
  +------------------------+  ~S6-1        +----------------------------+
  | Inputting OGTT glucose |               |  Generating template and   |
  |      OGTT insulin      |               |    registering databese    |
  |    Time-series data    |               +----------------------------+
  +------------------------+                            |
             |                                          v
             v
  +------------------------+  ~S6-2                  _____
  | Comparing between OGTT |                        /       \
  | time-series data and   |        Time-series    | DB3    |
  | each time-series data  |<- - - - -  data - - - | Template |
  | of database and        |                       | databese |
  | extracting template    |                        _____/
  | with minimum error     |                            |
  | summation used for     |                            |
  | similarity             |                            : (dotted)
  | determination          |                            :
  +------------------------+                            :
             |                                          :
             v                                          :
          /\  S6-3                                      :      S6-4
         /  \                          +--------------------------------+
        / similarity \     YES         | Obtaining parameter set        |
       / determination \-------------->| corresponding to time-series   |
       \ value of the  /               | data of template having the    |
        \ extracted   /                | highest matching rate from     |
         \ template  /                 | template databese              |
          \ is lower /                 +--------------------------------+
           \ than   /                                  |
           threshold                                   v
            value or                           +--------------------+  S6-5
              not?                             | Estimating biological |
             \/                                | mathematical model    |
             | NO                              | parameter set with    |
             |                                 | default parameter set |
             |                                 | search range          |
             |                                 +--------------------+
             |                                          |
             v  S6-7                                    v  S6-6
  +------------------------+           +--------------------------------+
  | Estimating process of  |           | Selecting parameter set within |
  | biological mathematical|           | parameter selection range      |
  | model parameter set    |           +--------------------------------+
  | with default parameter |                          |
  | set search range       |                          |
  +------------------------+                          |
             |                                         |
             +------------------+----------------------+
                                |
                                v
                    +------------------------+  S6-8
                    | Outputting biological  |
                    |    function profile    |
                    +------------------------+
                                |
                                v
                             ( END )
```

European Patent
Office

**EUROPEAN SEARCH REPORT**

Application Number

EP 06 00 9780

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/172230 A1 (SCHMITT W; WILLMANN S; SCHMITT) 2 September 2004 (2004-09-02) | 1-4,7 | INV.<br>G09B23/28 |
| Y | * paragraphs [0010], [0023], [0029]; figures 2,3 * | 5,6 | G06F17/50<br>G06F19/00 |
| Y | US 2005/071141 A1 (BUTLER RICHARD) 31 March 2005 (2005-03-31) | 5,6 | |
| A | * the whole document * | 1-4,7 | |
| Y | BARRETT P H R ET AL: "SAAM II: Simulation, analysis, and modeling software for tracer and pharmacokinetic studies" METABOLISM, CLINICAL AND EXPERIMENTAL, W.B. SAUNDERS CO., PHILADELPHIA, PA, US, vol. 47, no. 4, April 1998 (1998-04), pages 484-492, XP004538736 ISSN: 0026-0495 | 5,6 | |
| A | * the whole document * | 1-4,7 | |
| A | RICCARDO BELLAZZI ET AL: "Closed-Loop and Partially Closed-Loop Control Strategies for Insulin Delivery and Measuring Glucose Concentration" IEEE ENGINEERING IN MEDICINE AND BIOLOGY MAGAZINE, IEEE SERVICE CENTER, PISACATAWAY, NJ, US, vol. 20, no. 1, January 2001 (2001-01), pages 54-64, XP011085089 ISSN: 0739-5175 * the whole document * | 1-7 | TECHNICAL FIELDS SEARCHED (IPC)<br><br>G06F<br>G06G<br>G09B |
| A,P | EP 1 580 682 A (SYSMEX CORPORATION) 28 September 2005 (2005-09-28) * the whole document * | 1-7 | |
| A | US 2003/033126 A1 (LINCOLN PATRICK DENIS ET AL) 13 February 2003 (2003-02-13) * the whole document * | 1-7 | |

The present search report has been drawn up for all claims

| Place of search | Date of completion of the search | Examiner |
|---|---|---|
| Munich | 3 August 2006 | Wellisch, J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 00 9780

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2006

| Patent document cited in search report | | Publication date | Patent family member(s) | | Publication date |
|---|---|---|---|---|---|
| US 2004172230 | A1 | 02-09-2004 | CA DE EP JP | 2451501 A1<br>10256315 A1<br>1426763 A1<br>2004238390 A | 03-06-2004<br>17-06-2004<br>09-06-2004<br>26-08-2004 |
| US 2005071141 | A1 | 31-03-2005 | CA EP WO GB JP | 2459673 A1<br>1428165 A2<br>03023682 A2<br>2381907 A<br>2005502406 T | 20-03-2003<br>16-06-2004<br>20-03-2003<br>14-05-2003<br>27-01-2005 |
| EP 1580682 | A | 28-09-2005 | CN JP US | 1670755 A<br>2005267042 A<br>2005234311 A1 | 21-09-2005<br>29-09-2005<br>20-10-2005 |
| US 2003033126 | A1 | 13-02-2003 | WO | 03084465 A2 | 16-10-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BERGMAN.** *American Journal of Physiology,* 1979, vol. 236-6, E-667-77

- *Journal of Clinical Investigation,* 1981, vol. 68 (6), 1456-67